# EUROPÄISCHE PATENTSCHRIFT

(11) **EP 0 576 842 B1**
(45) Veröffentlichungstag und Bekanntmachung des Hinweises auf die Patenterteilung: **16.10.2002**
(21) Anmeldenummer: 93108775.3
(22) Anmeldetag: 01.06.1993
(51) Int. Cl.: C12Q 1/68, C07K 14/195, G01N 33/569

(54) **Verfahren und Mittel zum Nachweis von Listerien**
Method and means for detecting listeria
Méthode et moyens pour détecter listeria

(30) Priorität: 11.06.1992 DE 4219111; 25.11.1992 DE 4239567
(43) Veröffentlichungstag der Anmeldung: 05.01.1994
(73) Patentinhaber: MERCK PATENT GmbH, 64271 Darmstadt (DE)
(72) Erfinder: Goebel, Werner, Prof.Dr., W-8707 Veitshöchheim (DE); Bubert, Andreas, W-8708 Gerbrunn (DE); Köhler, Stefan, Dr., F-34980 St. Clément (FR); Pawelzik, Martina, Dr., D-81739 München (DE); Hofmann, Gottfried, Dr., W-6100 Darmstadt (DE); Neumann, Siegfried, Dr., W-6100 Seeheim-Jugenheim (DE); Schubert, Peter, Dr., W-6100 Darmstadt (DE); Linxweiler, Winfried, Dr., W-6114 Gross-Umstadt (DE); Burger, Christa, Dr., W-6100 Darmstadt (DE)

(56) Entgegenhaltungen:
- WO-A-90/10870
- FR-A- 2 616 804
- INFECTION AND IMMUNITY, Bd.58, Nr.5, Mai 1990 Seiten 1943 - 1950 KÖHLER ET AL. 'The gene coding for protein p60 of Listeria monocytogenes ....'
- APPLIED AND ENVIRONMENTAL MICROBIOLOGY, Bd.54, Nr.12, Dezember 1988, WASHINGTON, DC Seiten 2933 - 2937 DATTA ET AL. 'Synthetic oligodeoxyribonucleotide probes ....'
- LETTERS IN APPLIED MICROBIOLOGY, Bd.11, 1990, OXFORD Seiten 158 - 162 BORDER ET AL. 'Detection of Listeria species ....'
- APPLIED AND ENVIRONMENTAL MICROBIOLOGY, Bd.58, Nr.8, August 1992, WASHINGTON, DC. Seiten 2625 - 2632 BUBERT ET AL. 'The homologous and heterologous regions within the iap gene allow ....'

## Beschreibung

Die Erfindung betrifft Mittel und Verfahren zum Nachweis von Bakterien der Gattung Listeria, insbesondere von L. monocytogenes.

Die Mitglieder der Gattung Listeria sind grampositive stäbchenförmige Bakterien, die ubiquitär vorkommen. Zu dieser Gattung gehören sieben verschiedene Arten: L. monocytogenes, L. ivanovii, L. seeligeri, L. welshimeri, L. innocua, L. murrayi und L. grayi. Von diesen ist nur L. monocytogenes pathogen für Menschen, gefährdet sind insbesondere Neugeborene, Schwangere und Ältere, sowie Patienten unter Immunsuppression. Häufig verlaufen Infektionen mit L. monocytogenes tödlich.

Die Ursache für Infektionen mit Listerien sind häufig kontaminierte Lebensmittel, in denen sich die Keime selbst bei niedrigen Temperaturen (um 4°C) vermehren können. So wurden verschiedene Listerien-Epidemien auf den Verzehr von kontaminierten Nahrungsmitteln, wie z.B Rohmilch, Käse oder Krautsalat, zurückgeführt. Schnelle Nachweisverfahren für den Nachweis von Listerien, insbesondere in Lebensmitteln oder klinischen Proben, sind also dringend erforderlich. Diese Verfahren müssen zudem zwischen L. monocytogenes und den nicht-humanpathogenen Arten unterscheiden können. Weiterhin ist erforderlich, daß alle Varianten der humanpathogenen Art L. monocytogenes nachgewiesen werden können.

In jüngster Zeit wird in der Fachwelt diskutiert, ob die Listerienart L. innocua als Leitkeim für das mögliche Vorkommen von L. monocytogenes sinnvoll eingesetzt werden könnte. Deswegen wäre auch ein Nachweis von L. innocua nützlich.

Der Nachweis von L. monocytogenes erfolgt bisher mittels Verfahren, die auf der Kultur der Mikroorganismen beruhen. Das in Int. J. Food Microbiol. **4** (1987), 249-256 beschriebene Verfahren dauert zwei Wochen. Ein etwas schnelleres Verfahren wird von der International Dairy Foundation (IDF) empfohlen; es dauert aber mindestens 6-8 Tage. Beide Verfahren sind wegen ihrer Dauer für eine schnelle Identifizierung ungeeignet. Beide Verfahren sind zudem arbeitsintensiv, da für die Gewinnung von Einzelkolonien Nährmedien mehrfach inokuliert werden müssen, und da anschließend die Isolate mittels biochemischer und serologischer Untersuchungsmethoden charakterisiert werden müssen.

Die bisher auf dem Markt befindlichen immunologischen Tests dauern zwar nur wenige Stunden, erlauben jedoch nicht die wichtige Unterscheidung zwischen verschiedenen Arten von Listerien. Auch bei diesen Verfahren wird eine zweitägige Voranreicherungskultur benötigt.

In Appl. Environ. Microbiol. **54** (1988), 2933-2937 ist eine Methode beschrieben, bei der L. monocytogenes mit Hilfe von synthetischen Oligodesoxyribonukleotid-Sonden spezifisch nachgewiesen wird. Jedoch sind die verwendeten Sonden nicht ausreichend spezifisch, da sie auch mit der nicht human-pathogenen Art L. seeligeri reagieren. Auch für dieses Verfahren ist eine vorherige Vermehrung der Keime notwendig: Lebensmittelproben bzw. deren Verdünnungen werden auf Agarplatten ausgestrichen, die beimpften Platten werden bebrütet und anschließend mit einer radioaktiv markierten DNS-Sonde im colony Hybridisierungsverfahren untersucht. Der Nachweis erfolgt durch Autoradiographie. Auch diese Methode ist arbeits- und zeitaufwendig.

In Infect. Immun. 58, 1943-1950 (1990) wird die DNA-Sequenz des iap-Gens (invasion-associated protein) von L. monocytogenes beschrieben. Dieses Gen kodiert ein Protein, das auch unter der Bezeichnung p60 bekannt ist, und das in Varianten in allen Listeria Arten vorkommt. Bei L. monocytogenes ist dieses Protein für die Fähigkeit der Bakterien, in tierische Zellen einzudringen, verantwortlich. Ein Polynukleotid (400 Basen) mit einer Teilsequenz aus diesem Gen ist als DNA-Sonde geeignet, um L. monocytogenes von anderen Organismen zu unterscheiden.

Die Polymerase-Ketten-Reaktion (Polymerase-Chain-Reaction: PCR) erlaubt die in-vitro Vermehrung von Nukleinsäuren, eine Vorkultur ist bei diesem Verfahren im allgemeinen nicht notwendig. Damit die Reaktion gestartet werden kann, werden kurze Nukleinsäurestücke (primer) benötigt, die den zu vermehrenden Genomabschnitt eingrenzen. Üblicherweise werden zwei Primer benutzt; die mit jeweils einem Nukleinsäurestrang hybridisieren. Einer der Primer besitzt deswegen die Komplementärsequenz des jeweiligen Genabschnittes. Die ' Auswahl dieser Primer bestimmt die Spezifität der Nachweisreaktion. Für den Nachweis von L. monocytogenes ist dieses Verfahren in Appl. Environmental Microbiology **57**, 606-609 (1991), in Letters Appl. Microbiol. **11,** 158-162 (1990) und in J. Appl. Bact. **70**, 372-379 (1991) beschrieben. Dort finden sich nähere Angaben zu den Einzelheiten dieser Verfahren. Die DNA-Primer binden an das Gen für Listeriolysin, dem Listeria- Hämolysin. Die Spezifität dieser Primer ist, wie aus Anmerkungen in J. Appl. Bact. **70** hervorgeht, zumindestens unsicher: L. seeligeri läßt sich nicht sicher von L. monocytogenes unterscheiden. Mit Hilfe der bisher beschriebenen Primer in der PCR-Technik ist somit der sichere Nachweis von L. monocytogenes bisher nicht möglich.

Polyklonale Antikörper gegen L. monocytogenes p60 reagieren auch mit dem Protein p60 von anderen, apathogenen Listerienarten. Derartige Antikörper sind deswegen ungeeignet, L. monocytogenes durch immunologische Verfahren spezifisch nachzuweisen. Es ist zwar prinzipiell möglich, ein derartiges polyvalentes Antiserum durch spezifische Absorption von störenden Antikörperfraktionen zu reinigen: Dazu müßte gegebenenfalls Protein p60 von allen anderen Listeria-Arten an Träger kovalent gebunden werden. Die nicht erwünschten Antikörperfraktionen lassen sich spezifisch absorbieren; übrig bleibt ein Antiserum, das nur noch mit Protein p60 aus L. monocytogenes reagiert. Diese Methode zur Gewinnung eines für L. monocytogenes spezifischen Serums ist aufwendig: Man benötigt erhebliche Mengen von dem polyvalenten Antiserum als Ausgangsmaterial, sowie zusätzlich die iap-Genprodukte p60 von verschiedenen Listerienarten. Bei der Gewinnung von monoklonalen Antikörpern gegen Protein p60 würde dieser hohe Materialbedarf nicht auftreten, jedoch ist die Entstehung von Antikörpern gegen bestimmte Epitope zufallsbedingt: Zunächst muß eine große Zahl von antikörperproduzierenden Zellklonen hergestellt werden, aus denen dann Klone mit geeigneten Antikörpern ausgewählt werden müssen. Es ist bisher nicht möglich, gezielt Antikörper gegen Epitope zu gewinnen, die für L. monocytogenes spezifisch sind. Ähnliches gilt für Antikörper gegen Epitope, die für L. innocua spezifisch sind.

Aufgabe der vorliegenden Erfindung ist es, verbesserte Mittel und Methoden für die Differenzierung von Bakterien der Gattung Listeria, insbesondere für den Nachweis von Bakterien der Art L. monocytogenes bereitzustellen. Insbesondere werden erfindungsgemäß Peptide zur gezielten Erzeugung von spezifischen Antikörpern, die für den artspezifischen immunologischen Nachweis von Bakterien der Art L. monocytogenes geeignet sind, bereitgestellt.

Gegenstand der Erfindung sind Peptide, die als Teilsequenz mindestens eine Sequenz nach einer der Formeln IIIa oder IIId enthalten, wobei vor und/oder hinter dieser Teilsequenz bis zu jeweils sieben Aminosäuren peptidisch gebunden vorliegen können.

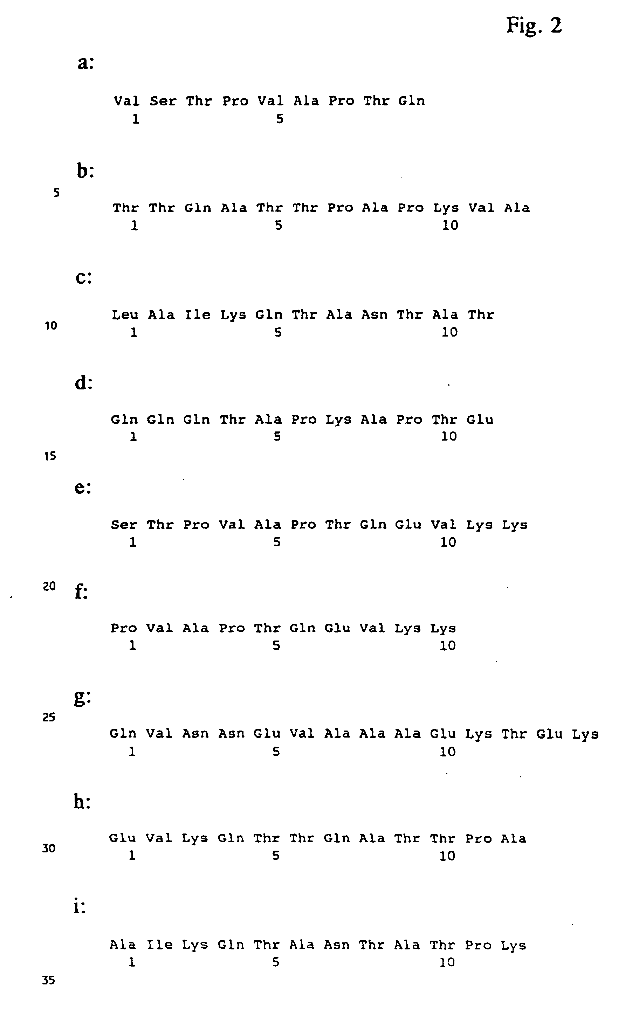

Besonders bevorzugt sind dabei Peptide mit einer Sequenz nach einer der Abbildungen 2 a, 2 d, 2 e oder 2 f[SeqID 26, SeqID29, SeqID 30 oder SeqID 31].

Gegenstand der Erfindung ist auch die Verwendung eines der genannten Peptide mit einer Teilsequenz nach einer der Formeln IIIa oder IIId zur Herstellung von immunogenen Konjugaten. Besonders bevorzugt sind für diesen Verwendungszweck Peptide mit einer Sequenz nach einer der Abbildungen 2 a, 2 d, 2 e oder 2 f [SeqID 26, SeqID29, SeqID 30 oder SeqID 31].

Gegenstand der Erfindung ist auch ein Antikörper, der ein Epitop bindet, das ein Peptid nach einer der Abbildungen 2 a, 2 d, 2 e oder 2 f [SeqID 26, SeqID29, SeqID 30 oder SeqID 31] enthält.

Gegenstand der Erfindung ist auch ein Verfahren zur Herstellung eines Antikörpers gerichtet gegen das Protein p60 aus Listerien, indem man ein Versuchstier mit einem Immunogen immunisiert und die Antikörper isoliert, dadurch gekennzeichnet, daß man als Immunogen ein immunogenes Konjugat verwendet, das ein Peptid nach einer der Formeln IVa oder IVd enthält, worin X³ und X⁴ jeweils unabhängig voneinander Wasserstoff, eine beliebige Aminosäure oder ein beliebiges Oligopeptid mit bis zu 7 Aminosäuren bedeuten.

Besonders bevorzugt sind dabei Peptide mit einer Sequenz nach einer der Abbildungen 2 a, 2 d, 2 e oder 2 f [SeqID 26, SeqID 29, SeqID 30 oder SeqID 31].

Gegenstand der Erfindung ist weiterhin die Verwendung eines Antikörpers, der gegen eines der Epitope mit einer Aminosäuresequenz nach einer der Abbildungen 2 a, 2 d, 2 e oder 2 f [SeqID 26, SeqID 29, SeqID 30 oder SeqID 31] gerichtet ist, für den Nachweis von Bakterien der Gattung Listeria.

Gegenstand der Erfindung sind auch Verfahren zum Nachweis von Bakterien der Gattung Listeria mittels eines Antikörpers, der gegen eines der Epitope mit einer Aminosäuresequenz nach einer der Abbildungen 2 a, 2 d, 2 e oder 2 f [SeqID 26, SeqID 29, SeqID 30 oder SeqID 31] gerichtet ist.

Gegenstand der Erfindung sind ferner Testzusammenstellungen zum immunologischen Nachweis von Bakterien der Art Listeria monocytogenes, in denen ein Antikörper, der gegen eines der Epitope mit einer Aminosäuresequenz nach einer der Abbildungen 2 a, 2 d, 2 e oder 2 f [SeqID 26, SeqID 29, SeqID 30 oder SeqID 31] gerichtet ist, enthalten ist.

Figur 1 zeigt das Ergebnis der elektrophoretischen Auftrennung von Amplifikationsprodukten; die experimentellen Einzelheiten sind in Beispiel 8 dargestellt.

Figur 2 a-i [SeqID 26-34] zeigen die Aminosäuresequenzen der besonders bevorzugten immunogenen Peptide ausgewählt aus der Sequenz des Proteins p60 aus Listeria monocytogenes.

Figur 3 zeigt die Aminosäuresequenz des Polypeptides ausgewählt aus der Sequenz des Proteins p60 aus Listeria monocytogenes [SeqID 39], dessen Epitope für den immunologischen Nachweis von Bakterien der Gattung Listeria geeignet sind.

Figur 4 zeigt für Vergleichszwecke die Aminosäuresequenz des Proteins p60 aus Listeria monocytogenes [SeqID 40], die in zwei Teilabbildungen a und b dargestellt ist.

Figur 5 a-d zeigen die Aminosäuresequenzen der besonders bevorzugten immunogenen Peptide ausgewählt aus der Sequenz des Proteins p60 aus Listeria innocua [SeqID 35-38].

Die Erfindung wird im folgenden näher beschrieben. Dabei wird in der Regel auf Einzelheiten von biochemischen, immunologischen und molekularbiologischen Verfahren, die dem Fachmann bekannt sind, und deren Einzelheiten in der Literatur beschrieben sind, nicht näher eingegangen. Bei diesen Verfahren kann man auch von an sich bekannten, hier nicht näher beschriebenen Varianten Gebrauch machen.

Die erfindungsgemäßen Oligonukleotide nach den Formeln Ia - Ih [SeqID 1-8] und IIa - IIh [SeqID 9-16] sind als Primer für Nukleinsäureamplifikationsmethoden und somit zum spezifischen Nachweis von Bakterien der Gattung Listeria geeignet. Ihre Sequenzen sind in üblicher Weise vom 5'-Ende zum 3'-Ende geschrieben dargestellt. In Abhängigkeit von den Erfordernissen des jeweils verwendeten Amplifikationssystems werden entweder Desoxyribonukleotide oder auch Ribonukleotide mit den erfindungsgemäßen Sequenzen eingesetzt. Im letzteren Fall sind die Thymidinbausteine jeweils durch Uridinbausteine ersetzt. Dem Fachmann ist weiterhin bekannt, daß häufig der Austausch von einer oder weniger Basen in einer Nukleotidsequenz deren biologische Eigenschaften nicht verändert. Deswegen umfassen die erfindungsgemäßen Nukleotidsequenzen auch solche, die durch Basenaustausch aus den Sequenzen Ia - Ih und IIa - IIh abgeleitet sind, und die biologisch dieselbe Wirkung wie der jeweilige Primer mit der Ursprungssequenz zeigen. Da üblicherweise je ein Primer mit jeweils einem der DNA-Stränge reagieren soll, wird einer der Primer in der komplementären Sequenz eingesetzt. Die komplementäre Sequenz ergibt sich in bekannter Weise nach den Regeln der Basenpaarung.

Basierend auf der jeweiligen Sequenz können die erfindungsgemäßen Oligonukleotide nach dem Fachmann bekannten Verfahren, beispielsweise der Phosphotriester- oder der Phosphoamidit-Methode, synthetisiert werden. Bevorzugt wird die Phosphoamidit-Methode benutzt, insbesondere unter Verwendung von mechanisierten Synthetizern. Die Methode ist in Tetrahedron Lett. (1981) **22**: 1859-1862 beschrieben. Weitere Einzelheiten derartiger Syntheseverfahren sind beispielsweise in Winnacker, E.L. (1985) Gene und Klone, Seite 44-61 (VCH-Verlagsgesellschaft mbH, Weinheim), beschrieben.

Die erfindungsgemäßen Primer sind geeignet für DNS-Amplifikation, beispielsweise mit Hilfe der Polymerase Chain Reaction (PCR). Dazu wird die DNS durch Erwärmen zunächst in die Einzelstränge zerlegt. Es werden zwei Primer verwendet, die jeweils mit dem homologen DNS-Abschnitt auf jeweils einem DNS-Strang hybridisieren. Der Genomabschnitt, der zwischen diesen beiden Primern liegt, wird vermehrt. Die an die DNS angelagerten Primer stellen die Startpunkte für die Amplifikation dar. Eine Polymerase, bevorzugt taq-DNA-Polymerase, ergänzt anschließend in Gegenwart der vier Nukleotidtriphosphate den zweiten Strang entsprechend der Sequenz der ursprünglichen DNA. Anschließend werden die entstandenen Doppelstränge wieder durch Erwärmen in die Einzelstränge zerlegt. Dieser Amplikationszyklus kann mehrfach wiederholt werden. Nach einer ausreichenden Anzahl von Amplifikationszyklen kann die amplifizierte Nukleinsäure mittels bekannter Methoden nachgewiesen werden. Dazu kann die DNS mittels Elektrophorese aufgetrennt, anschließend mit Ethidiumbromid angefärbt und schließlich durch Fluoreszenz mittels UV-Anregung nachgewiesen werden. Möglich ist auch der Nachweis mittels DNS-Hybridisierung. Die Einzelheiten geeigneter Amplifikations- und Nachweismethoden sind auch in Übersichtsartikeln, z.B. Innis et al. (eds.) PCR Protocols (Academic Press, Inc., Harcourt Brace Jovanovich, Publishers) beschrieben. Ebenso sind andere Nukleinsäureamplifikationsverfahren, bei denen die erfindungsgemäßen Primer benutzt werden können, aus der Literatur bekannt. Zu diesen gehört die Ligase-Ketten-Reaktion, beschrieben von Bond, S. et al. (1990), Seite 425-434 bei Raven Press (New York, NY/USA).

Die Auswahl der Primer nach den bevorzugten Formeln IIa - IIh bestimmt die Lage der Startpunkte auf dem iap-Gen und somit die Spezifität der Nachweisreaktion: So erwiesen sich Kombinationen von Primern ausgewählt aus der Sequenz des iap-Gens als unspezifisch und folglich ungeeignet für den Nachweis von Listerien mittels DNA-Amplifikation (siehe dazu beispielsweise Spalte F in Tabelle 1). Jedoch erwiesen sich andere ausgewählte Kombinationen als spezifisch für die Gattung Listeria, andere für Gruppen von Listeria-Arten, wieder andere für einzelne Listeria-Arten. Insgesamt ist die Auswahl und die Zusammenstellung der Primer also kritisch. Die Auswahl eines der beiden Primer ist stets besonders kritisch, während der zweite Primer eher variiert werden kann, ohne die Spezifität der Nachweisreaktion nennenswert zu verändern. Folglich kann nach der Lehre der vorliegenden Erfindung für diesen zweiten Primer durchaus auch eine Sequenz gewählt werden, die nicht einer der Formeln Ia - Ih oder IIa - IIh entspricht.

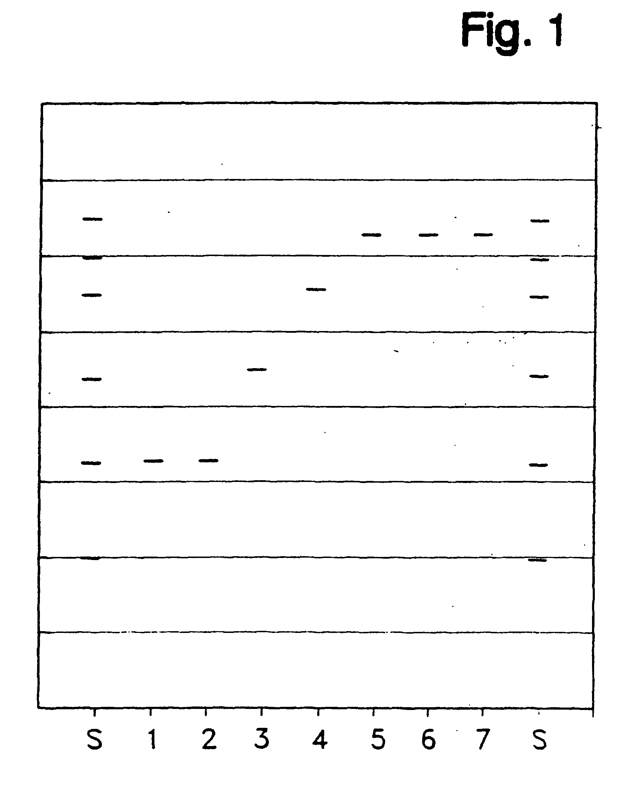

Erfindungsgemäß wird zumindestens einer der Primer aus den Formeln Ia - Ih oder bevorzugt aus den Formeln IIa - IIh ausgewählt. Der zweite Primer beeinflußt, wie bereits erläutert, die Spezifität der Amplifikationsreaktion wesentlich weniger als der erste Primer. Bevorzugt werden jedoch Kombinationen, bei denen beide Primer aus den Formeln Ia - Ih oder IIa - IIh ausgewählt werden. Beispiele für derartige bevorzugte Kombinationen sind (typische Ergebnisse sind in Tabelle 1 zusammengefaßt):
a) Bei Verwendung einer Kombination eines Primers nach Formel IIc mit einem Primer mit der Komplementärsequenz nach Formel IIb wird nur die DNS von Listerien amplifiziert, nicht jedoch die DNS von anderen Bakterienarten (siehe Spalte D in Tabelle 1).
b) Bei Verwendung einer Kombination eines Primers nach Formel IId mit einem Primer mit der Komplementärsequenz nach Formel IIb wird nur die DNS von L. monocytogenes amplifiziert, nicht jedoch die DNS von anderen Listerien oder anderen Bakterien (siehe Spalte B in Tabelle 1).
c) Bei Verwendung einer Kombination eines Primers nach Formel IIf mit einem Primer mit der Komplementärsequenz nach Formel IIb wird nur die DNS von bestimmten Listerien-Arten amplifiziert, nämlich ausschließlich von L. seeligeri, L. welshimiri und L. ivanovii. Mithin ist ein gruppenspezifischer Nachweis möglich (siehe Spalte E in Tabelle 1).
d) Ein anderes Beispiel für einen gruppenspezifischen Nachweis besteht in der Verwendung einer Kombination eines Primers nach Formel IIh mit einem Primer mit der Komplementärsequenz nach Formel IIb: Es wird nur die DNS von L. grayi und L. murrayi amplifiziert (siehe Spalte G in Tabelle 1).
e) Da die Amplifikationsprodukte verschiedener Listerien-Arten unterschiedliche Molekulargewichte aufweisen, können durch eine Kombination von mehreren Primern (nach Formeln IId, IIf, IIg und IIh) mit der Komplementärsequenz von Formel IIb mit einer einzigen Polymerasereaktion Bakterien der Gattung Listeria differenziert werden. Einzelheiten dieser Weiterentwicklung der Polymerasetechnik sind aus Beispiel 8 ersichtlich (siehe Spalte H in Tabelle 1, sowie Figur 1).

Wie bereits erwähnt, ist es auch möglich, die Amplifikationsprodukte durch Nukleinsäurehybridisierung nachzuweisen. Dazu werden dem Reaktionsansatz nach der Amplifikation geeignete Nukleinsäurestücke (Nukleinsäuresonden) zugesetzt. Diese Nukleinsäuresonden besitzen eine Basensequenz, die ganz oder teilweise komplementär zu dem amplifizierten Genabschnitt ist. Diese Sonden sind außerdem für eine Nachweisreaktion markiert: Sie können radioaktive Isotope enthalten, oder Fluoreszenzmarkierungen tragen, oder auch durch Enzyme markiert sein. Geeignete Markierungsmittel, Methoden zu ihrer Einfügung in die Nukleinsäuresonde und Nachweismethoden sind dem Fachmann bekannt.

Im besonderen kann die Amplifikationsreaktion spezifisch für die Gattung Listeria (wie oben unter a) näher beschrieben) oder für eine Gruppe von Listerien-Arten (wie oben unter c) und d) beschrieben) ausgelegt sein. Durch Verwendung von Nukleinsäuresonden, die fiir jeweils eine Art spezifisch sind, kann dann im Reaktionsansatz, das Vorhandensein dieser Arten von Listerien erkannt werden. Wenn die Sonden verschiedene Markierungsmittel enthalten, können auch verschiedene Arten nebeneinander nachgewiesen werden. Diese Verfahrensvariante erlaubt also ähnlich der unter e) oben beschriebenen den Nachweis verschiedener Listerien-Arten nebeneinander.

Die Verwendung einer Nukleinsäuresonde oder eines Gemisches verschiedener Sonden, die mit Amplifikationsprodukten aller Listerienarten reagieren, erlaubt es, die Spezifität der Amplifikationsreaktion zu überprüfen oder ein einheitliches Nachweisreagenz für verschiedene Listerien-Arten bereitzustellen.

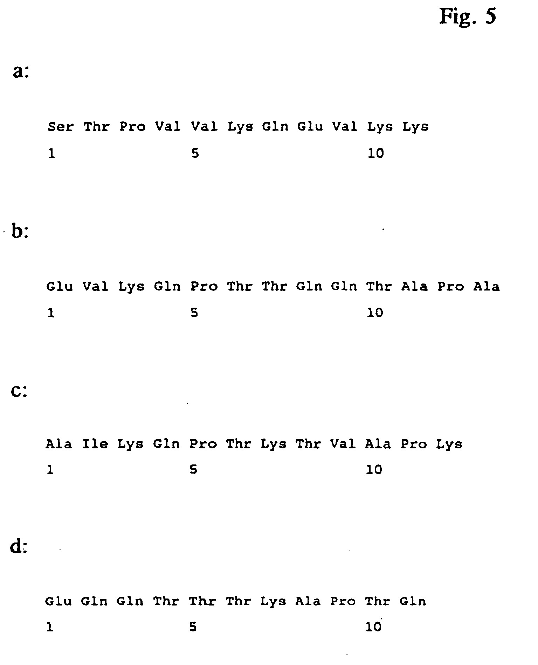

Die erfindungsgemäßen Peptide nach den Formeln IVa - IVi [SeqID 17-25] und nach Abbildung 2 a-i [SeqID 26-34], sowie nach Abbildung 5 a-d [SeqID 35-38] können in immunogene Konjugate eingebaut werden. Mit Hilfe dieser Konjugate können Antikörper erzeugt werden, die es ermöglichen, Bakterien der Gattung Listeria mit immunologischen Methoden spezifisch nachzuweisen.

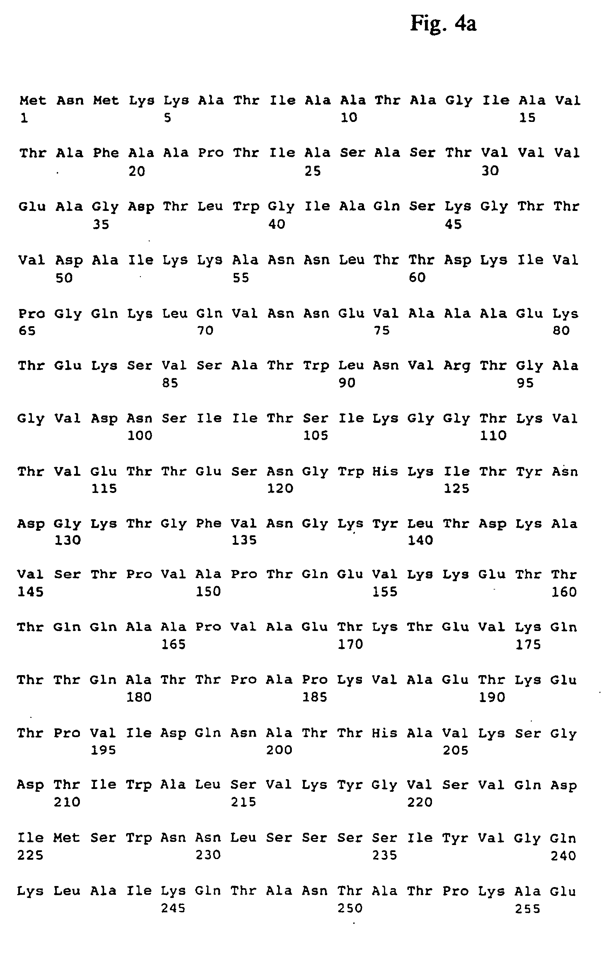

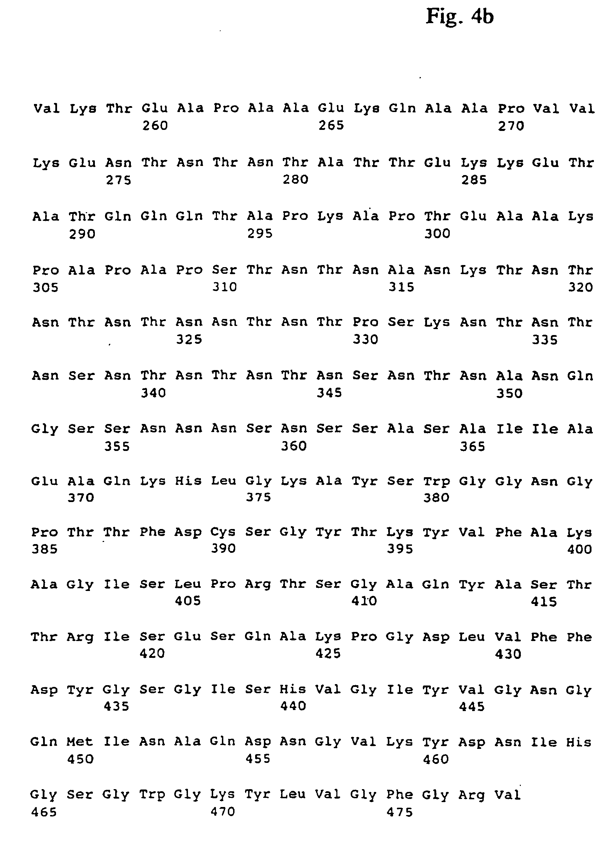

Die Positionen der erfindungsgemäßen Peptide in der Gesamtsequenz des Proteins p60 aus Listeria monocytogenes sind im folgenden angegeben:
a) Die Sequenz nach Formel IIIa beginnt mit Prolin an Position 148 der p60 Sequenz (Fig. 4a); in dieser Region befinden sich auch die Peptide nach Fig. 2a, 2e und 2f.
b) Die Sequenz nach Formel IIIb beginnt mit Threonin an Position 178 der p60 Sequenz (Fig. 4a); in dieser Region befinden sich auch die Peptide nach Fig. 2b und 2h.
c) Die Sequenz nach Formel IIIc beginnt mit Alanin an Position 243 der p60 Sequenz (Fig. 4a); in dieser Region befinden sich auch die Peptide nach Fig. 2c und 2i.
d) Die Sequenz nach Formel IIId beginnt mit Glutamin an Position 292 der p60 Sequenz (Fig. 4b); in dieser Region befindet sich auch das Peptid nach Fig. 2d.
e) Die Sequenz nach Formel IIIe beginnt mit Valin an Position 71 der p60 Sequenz (Fig. 4a); in dieser Region befindet sich auch das Peptid nach Fig. 2g.

Die Sequenzen der erfindungsgemäßen Peptide nach Abbildung 5 a-d stammen aus der Gesamtsequenz des Proteins p60 aus Listeria innocua; gleiches gilt für die mit den Formeln IIIf- IIIi und IVf- IVi beschriebenen Teilsequenzen.

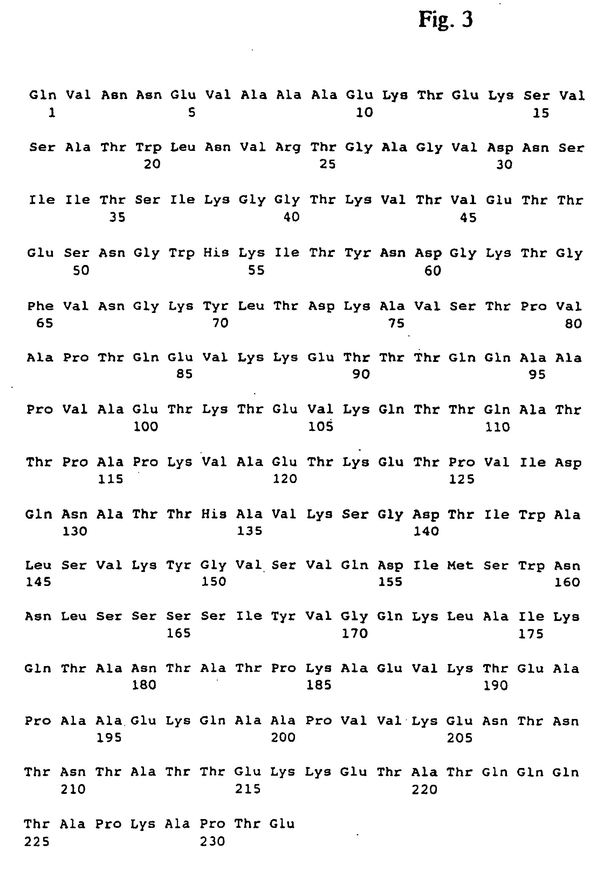

Dem Fachmann ist bekannt, daß häufig der Austausch von einer oder wenigen Aminosäuren in einem Peptid dessen biologische Eigenschaften nicht verändert. Deswegen umfassen die erfindungsgemäßen Peptidsequenzen auch solche, die durch Aminosäureaustausch aus den Sequenzen nach Fig. 2 a-i, nach Fig. 5 a-d oder nach Fig. 3 abgeleitet sind, und die biologisch dieselbe Wirkung wie die jeweiligen Peptide mit der Ursprungssequenz zeigen.

Die Auswahl der erfindungsgemäßen Peptide erwies sich als kritisch. Wählt man beispielsweise ein bestimmtes Peptid, das von dem für L. monocytogenes spezifischen Genabschnitt kodiert wird, für die Erzeugung von Antikörpern aus, so zeigt überraschenderweise keines der Antiseren eine Reaktion mit dem p60-Protein.

Basierend auf der Sequenz der Aminosäuren können die Peptide nach dem Fachmann bekannten Verfahren, beispielsweise dem tboc- oder dem fmoc-Verfahren (tert.butyloxycarbonyl-, bzw. 9-fluorenylmethyloxycarbonyl-), synthetisiert werden. Einzelheiten dieser Verfahren sind beispielsweise in J.Am.Chem.Soc.**85**, 2149-2154 (1963) und in Synthetic Polypeptides as Antigens (van Regenmortel et al. (eds.) Elsevier 1988 (Band 19 der Buchreihe Laboratory Techniques in Biochemistry and Molecular Biology) beschrieben. Bevorzugt wird das f_{moc}-Verfahren, insbesondere mechanisierte Verfahrensvarianten. Einzelheiten des Verfahrens, sowie geeignete Aminosäureschutzgruppen sind dem Fachmann bekannt.

Peptide sind im allgemeinen nicht geeignet, Antikörper zu erzeugen. Werden Peptide jedoch an hochmolekulare Trägersubstanzen gekoppelt, so entstehen immunogene Konjugate. Die erfindungsgemäßen Peptide lassen sich mit bekannten Trägersubstanzen konjugieren. Dazu gehören Polyäthylenglykole, Serumalbumine, KLH (Keyhole Limpet Hemocyanin; Hämocyanin von Napfschnecken), Ovalbumin, Glucosedehydrogenase aus Bacillus megaterium und PPD (purified protein derivative of tuberculin). Bevorzugte Trägersubstanzen sind KLH und Glucosedehydrogenase aus B. megaterium.

Außerdem werden zusätzlich häufig Brückenverbindungen (linker) eingesetzt. Dies sind niedermolekulare organische Verbindungen mit mindestens zwei verknüpfbaren funktionellen Gruppen. Geeignete Verbindungen sind dem Fachmann bekannt; dazu gehören beispielsweise: 1,2-Diaminoethan, Bernsteinsäure, β-Alanin, 1,6-Diaminohexan, 6-Aminocapronsäure, Adipinsäure, Cystein. Bevorzugt wird Cystein als Linker eingesetzt, wobei dieser Aminosäurerest bereits bei der Synthese des Peptides eingebaut wird. Linker, die sowohl eine Amino- als auch eine Carboxylfunktion enthalten (z.B. β-Alanin, 6-Aminocapronsäure oder Cystein), können wahlweise am C-oder N-Terminus des Peptids angeknüpft werden. Zur Herstellung der Bindungen zwischen Peptid und Trägersubstanz werden bevorzugt m-Maleimidobenzoyl-N-hydroxysuccinimidester (MBS) eingesetzt.

Die genannten immunogenen Konjugate dienen dazu, nach bekannten Verfahren in Versuchstieren Antikörper zu erzeugen. Üblicherweise werden für diesen Zweck Säugetiere, beispielsweise Schaf, Ziege, Kaninchen oder Mäuse benutzt. Kaninchen sind für die Erzeugung polyklonaler Antikörper bevorzugt. Es ist aber auch möglich, mit Hilfe der erfindungsgemäßen immunogenen Konjugate monoklonale Antikörper herzustellen.

Einzelheiten der immunologischen Verfahren sind dem Fachmann bekannt. Hinweise zur Durchführung dieser Verfahren sind außerdem vielfältig in der Literatur anzutreffen; beispielsweise seien genannt:
∗ Antibodies, E. Harlow und D. Lane, Cold Spring Harbor (1988)
∗ Woodard, L.F. und Jasman, R.L. (1985) Vaccine **3**, 137-144
∗ Woodard, L.F. (1989) Laboratory Animal Sci **39**, 222-225
∗ Handbook of Experimental Immunology (1986) Weir, D.M. et al. eds.; Blackwell Scientific Publications, Oxford, GB

Zu diesen Verfahren gehören beispielsweise die Konjugations- und Immunisierungsverfahren, sowie die Herstellung und Aufreinigung von Antikörpern und auch immunologische Nachweisverfahren. Zu den immunologischen Nachweisverfahren, bei denen erfindungsgemäße Antikörper benutzt werden können, gehören bevorzugt Agglutinationsverfahren, immunometrische Nachweisverfahren, die Immuno-Blot-Verfahren und insbesondere die sandwich-(ELISA)-Verfahren.

Der Begriff Antikörper umfaßt erfindungsgemäß sowohl Immunglobuline als auch Antiseren. Es ist dem Fachmann weiterhin bekannt, daß häufig statt eines einzigen Antikörpers, der gegen ein einzelnes Epitop gerichtet ist, eine Mischung von verschiedenen Antikörpern mit verschiedener Spezifität benutzt werden kann. Dadurch resultieren Vorteile, insbesondere bezüglich der Nachweisempfindlichkeit. Dies trifft im besonderen für monoklonale Antikörper, aber auch für andere Antikörper zu, die gegen jeweils ein Epitop gerichtet sind. Entsprechend kann es vorteilhaft sein, mehrere Antikörper, die gegen verschiedene Peptidstrukturen nach den Formeln IIIa - IIIi oder nach einer der Abbildungen 3, 2 a-i oder 5 a-d gerichtet sind, für die erfindungsgemäße Verwendung und/oder die erfindungsgemäßen Verfahren zu kombinieren.

Einzelheiten der Herstellung der erfindungsgemäßen Primer und Peptide, sowie ihrer Verwendung sind aus den folgenden Beispielen ersichtlich. Weitere methodische Einzelheiten entnimmt der Fachmann der zitierten Literatur. Die Beispiele sollen den Gegenstand der Erfindung illustrieren und stellen keine Einschränkung der Erfindung dar.

### Beispiele:

### Beispiel 1: Herstellung des Primers nach Formel IIa

Der Primer nach Formel IIa wird mit dem DNA-Syntheziser 380A von Applied Biosystems nach der Phosphoamidit-methode hergestellt. Die Grundzüge der Methode ist in Tetrahedron Lett. (1981) 22 : 1859-1862, beschrieben. Weitere Einzelheiten finden sich in der Dokumentation des Geräteherstellers.

Entsprechend werden die Primer nach Formel IIc, IId, IIf, IIg und IIh hergestellt. Die Primer nach Formel IIb und IIe werden in der jeweiligen Komplementärsequenz(IIb: TTGGCTTCGGTCGCGTAGAATTCATA; IIe: GCACTTGAATTGCTGTTATTG) hergestellt.

### Beispiel 2: Durchführung der PCR-Reaktion zum artspezifischen Nachweis von L. monocytogenes

Eine bakterienhaltige Probe mit ca. 1 µg DNA wird in 50 µl Puffer (10 mM Tris-HCl pH 8,5; 1,5 mM MgCl₂ und 50 mM KCl) suspendiert und 5 Minuten auf 110 °C erhitzt. Anschließend werden Primer nach Formel IId und IIe (siehe Beispiel 1; je 0,4 µg), sowie 2,5 U Taq-Polymerase (Fa. Pharmacia), gelöst in Reaktionspuffer (10 mM Tris-HCl pH 8,5; 1,5 mM MgCl₂ und 50 mM KCI), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 30 Sekunden auf 55 °C (Bindungsphase) und für eine Minute auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Polyacrylamid-Gel (6%) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

Nur wenn DNA oder Zellen von L. monocytogenes in der Probe vorhanden sind, werden PCR-Produkte beobachtet (siehe Spalte A in Tabelle 1).

### Beispiel 3: Durchführung der PCR-Reaktion zum artspezifischen Nachweis von L. monocytogenes

Das in Beispiel 2 beschriebene Verfahren wird unter Verwendung der Primer nach Formel IId und IIb (siehe Beispiel 1) anstelle der Primer nach Formel IId und IIe wiederholt. Auch in diesem Fall werden nur PCR-Produkte beobachtet, wenn DNA oder Zellen von L. monocytogenes in der Probe vorhanden sind (siehe Spalte B in Tabelle 1).

### Beispiel 4: Durchführung der PCR-Reaktion zum genusspezifischen Nachweis von Bakterien der Gattung Listeria

Eine bakterienhaltige Probe mit ca. 1 µg DNA wird in 50 µl Wasser suspendiert und 5 Minuten auf 110 °C erhitzt. Anschließend werden Primer nach Formel IIc und IIb (siehe Beispiel 1; je 0,4 µg), sowie 2,5 U Taq-Polymerase (Fa. Pharmacia), gelöst in Reaktionspuffer (10 mM Tris-HCl pH 8,5; 1,5 mM MgCl₂ und 50 mM KCl), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 30 Sekunden auf 56 °C (Bindungsphase) und für zwei Minuten auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Agarose-Gel (1%) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

In diesem Fall werden PCR-Produkte beobachtet, wenn DNA oder Zellen von Bakterien der Gattung Listeria in der Probe vorhanden sind (siehe Spalte D in Tabelle 1).

### Beispiel 5: Durchführung der PCR-Reaktion zum gruppenspezifischen Nachweis von Listerien

Eine bakterienhaltige Probe mit ca. 1 µg DNA wird in 50 µl Wasser suspendiert und 5 Minuten auf 110 °C erhitzt. Anschließend werden Primer nach Formel IIf und IIb (siehe Beispiel 1; je 0,4 µg), sowie 2,5 U Taq-Polymerase (Fa. Pharmacia), gelöst in Reaktionspuffer (10 mM Tris-HCl pH 8,5; 1,5 mM MgCl₂ und 50 mM KCl), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 45 Sekunden auf 58 °C (Bindungsphase) und für eine Minute auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Agarose-Gel (1%) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

In diesem Fall werden nur PCR-Produkte beobachtet, wenn DNA oder Zellen von Bakterien aus der Gruppe L. ivanovii, L. seeligeri und L. welshimeri in der Probe vorhanden sind (siehe Spalte E in Tabelle 1).

### Beispiel 6: Durchführung der PCR-Reaktion zum artspezifischen Nachweis von L. innocua

Eine bakterienhaltige Probe mit ca. 1 µg DNA wird in 50 µl Puffer (10 mM Tris-HCl pH 8,5; 1,5 mM MgCl₂ und 50 mM KCl) suspendiert und 5 Minuten auf 110 °C erhitzt. Anschließend werden Primer nach Formel IIg und IIb (siehe Beispiel 1; je 0,4 µg), sowie 2,5 U Taq-Polymerase (Fa. Pharmacia), gelöst in Reaktionspuffer (10 mM Tris-HCl pH 8,5; 1,5 mM MgCl₂ und 50 mM KCl), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 60 Sekunden auf 62 °C (Bindungsphase) und für 45 Sekunden auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Agarose-Gel (1 %) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

Nur wenn DNA oder Zellen von L. innocua in der Probe vorhanden sind, werden PCR-Produkte beobachtet (siehe Spalte C in Tabelle 1).

### Beispiel 7: Durchführung der PCR-Reaktion zum gruppenspezifischen Nachweis von Listerien

Eine bakterienhatige Probe mit ca. 1 µg DNA wird in 50 µl Wasser suspendiert und 5 Minuten auf 110 °C erhitzt. Anschließend werden Primer nach Formel IIh und IIb (siehe Beispiel 1; je 0,4 µg), sowie 2,5 U Taq-Polymerase (Fa. Pharmacia), gelöst in Reaktionspuffer (10 mM Tris-HCl pH 8,5; 1,5 mM MgCl₂ und 50 mM KCl), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 45 Sekunden auf 56 °C (Bindungsphase) und für 45 Sekunden auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Agarose-Gel (1%) in einem Laufpuffer Tris-Borat (je 50 mM) mit EDTA (2,5 mM) aufgetrennt. Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

In diesem Fall werden nur PCR-Produkte beobachtet, wenn DNA oder Zellen von Bakterien aus der Gruppe L. grayi und L. murrayi in der Probe vorhanden sind (siehe Spalte G in Tabelle 1).

### Beispiel 8: Durchführung einer kombinierten PCR-Reaktion zum artspezifischen Nachweis von L. monocytogenes und von L. innocua und zum gruppenspezifischen Nachweis der Gruppen L.ivanovii / L.seeligeri / L.welshimeri und L.grayi / L.murrayi

Eine bakterienhaltige Probe mit ca. 1 µg DNA wird in 50 µl Puffer (10 mM Tris-HCl pH 8,5; 1,5 mM MgCl₂ und 50 mM KCl) suspendiert und 5 Minuten auf 110 °C erhitzt. Anschließend wird eine Mischung von Primern nach Formel IId, IIf, IIg, IIh, sowie IIb (siehe Beispiel 1; je 0,4 µg), sowie 2,5 U Taq-Polymerase (Fa. Pharmacia), gelöst in Reaktionspuffer (10 mM Tris-HCl pH 8,5; 1,5 mM MgCl₂ und 50 mM KCl), sowie jeweils 200 µM dGTP, dATP, dTTP und dCTP zugefügt (gesamtes Reaktionsvolumen 100 µl). Der erste Denaturierungsschritt dauert 3 Minuten bei 94 °C. Anschließend wird für 45 Sekunden auf 56 °C (Bindungsphase) und für eine Minute auf 72 °C (Elongationsphase) temperiert. Die folgenden Denaturierungsschritte (bei 94 °C) dauern 45 Sekunden. Nach 30 Reaktionszyklen wird ein abschließender Elongationsschritt (bei 72 °C) mit 5 Minuten Dauer ausgeführt.

Die PCR-Produkte werden in einem Polyacrylamid-Gel (4%) in einem Laufpuffer Tris-Borat (je 50 mM) mit EDTA (2,5 mM) aufgetrennt. Zusätzlich wird eine Nukleinsäuremischung (beispielsweise das Produkt aus der Spaltung von Sppl Phagen DNA mittels Restriktionsendonuklease EcoRI) als Molekulargewichtsstandard mitgeführt.

Anschließend werden die aufgetrennte PCR-Produkte durch Anfärbung mit Ethidiumbromid (0,1 mg/ml in Wasser) angefärbt und Bestrahlung mit UV-Licht (260 nm) sichtbar gemacht.

Das Vorhandensein von DNA oder Zellen von Bakterien aus der Art L. monocytogenes, der Art L. innocua, der Gruppe L. ivanovii / L. seeligeri / L. welshimeri oder der Gruppe L. grayi / L. murrayi kann auf Grund der unterschiedlichen Molekulargewichte differenziert werden (siehe Spalte H in Tabelle 1, sowie Figur 1).

### Beispiel 9: Synthese des Peptides CysGlnGlnGlnThrAlaProLysAlaProThrGlu

Für die Synthese des Peptides CysGlnGlnGlnThrAlaProLysAlaProThrGlu wird das f_{moc}-Verfahren (9-Fluorenylmethyloxycarbonyl-Schutzgruppe) benutzt. Dieses Peptid entspricht einem Peptid der Formel IVd mit einem zusätzlichen N-terminalen Cysteinrest als Linker. Für die Synthese wird ein Peptid-Synthetizer der Fa. Applied Biosystems benutzt, die Prozessparameter sind in der Gerätedokumentation enthalten.

Ein polymerer Träger mit 4-(2',4'-Dimethoxyphenylaminomethyl)-phenoxygruppen dient als Festphase. Die Aminosäuren werden als α-N-f_{moc}-Derivate eingesetzt. Soweit die Aminosäuren reaktive Seitengruppen enthalten, werden diese durch zusätzliche Schutzgruppen, die durch Trifluoressigsäurehydrolyse abspaltbar sind, maskiert. Die Peptidbindungen werden durch Aktivierung der Carboxylgruppen mittels Diisopropylcarbodiimid hergestellt. Die Reihenfolge der eingesetzten Aminosäurederivate ergibt sich aus der gewünschten Sequenz.

Im ersten Schritt des Synthesezyklus reagiert die Aminogruppe an der Festphase, d.h. im ersten Zyklus die Aminogruppen des 4-(2',4'-Dimethoxyphenylaminomethyl)-phenoxyrests des Trägers, in den folgenden Zyklen die α-Aminogruppe der zuletzt angefügten , Aminosäure, mit der Carboxylgruppe der nächsten Aminosäure, die als α-N-f_{moc}-Derivat, gegebenenfalls mit geschützten Seitenketten eingesetzt wird, und die durch Diisopropylcarbodiimid aktiviert wird. Nicht umgesetzte Aminosäurederivate werden mit Dimethylformamid ausgewaschen. Anschließend wird die f_{moc}-Gruppe durch Behandeln mit 20 % (V/V) Piperidin in Dimethylformamid abgespalten. Die übrigen Schutzgruppen bleiben bei dieser Reaktion unverändert. Mit der Entfernung der α-N-Schutzgruppe kann der nächste Reaktionszyklus beginnen. Nachdem die letzte Aminosäure entsprechend der vorgesehenen Sequenz zugefügt worden ist, werden die Schutzgruppen der Seitenketten und die Bindung zum Trägerharz durch saure Hydrolyse mittels Trifluoressigsäure gespalten. Das Peptid wird anschließend durch Hochdruckflüssigkeitschromatographie gereinigt.

Entsprechend der oben beschriebenen Vorgehensweise werden auch die übrigen Peptide mit den erfindungsgemäßen Sequenzen synthetisiert.

### Beispiel 10: Konjugation des Peptides CysGlnGlnGlnThrAlaProLysAlaProThrGlu mit Glucosedehydrogenase

a) Derivatisierung der Glucosedehydrogenase: 30. mg Glucosedehydrogenase aus Bacillus megatherium (Fa. Merck, Art.Nr. 13732) werden in 4 ml Natriumphosphatpuffer (50 mM; pH 8,0) gelöst. Zu 2,4 ml dieser Lösung werden 6,78 mg N-y-Maleimidobutyryloxysuccinimid (Fa. Calbiochem) gelöst in 50 µl Dimethylsulfoxid zugegeben und 30 Minuten bei Raumtemperatur stehen gelassen. Anschließend wird das überschüssige N-y-Maleimidobutyryloxysuccinimid durch Gelfiltration an PD-10 (Fa. Pharmacia) chromatographisch abgetrennt. Nach der Chromatographie erhält man 3,5 ml Lösung des aktivierten Trägerproteins mit einer Konzentration von 4,5 mg/ml.
b) Kopplung mit dem Peptid: Zu 1,1 ml der Lösung aus dem obigen Schritt werden 5,2 mg des Peptides, hergestellt nach Beispiel 9, gelöst in 1 ml Natriumphosphatpuffer (50 mM; pH 7,0) zugefügt und 3 Stunden bei Raumtemperatur stehen gelassen. Anschließend wird das nicht gebundene Peptid durch Gelfiltration an PD-10 (Fa. Pharmacia) chromatographisch abgetrennt. Nach der Chromatographie erhält man 3,5 ml Lösung des Konjugates mit einer Konzentration von 2,3 mg/ml.

Entsprechend der oben beschriebenen Vorgehensweise werden auch Konjugate mit den anderen Peptiden entsprechend der vorliegenden Erfindung hergestellt.

### Beispiel 11: Erzeugung von polyklonalen Antikörpern gegen das Peptid CysGlnGlnGlnThrAlaProLysAlaProThrGlu

Zwei Kaninchen werden jeweils mit einer Emulsion aus 0,18 ml Konjugat aus Beispiel 10, 0,07 ml phosphatgepufferter Saline und 0,25 ml Öladjuvans (MISA 50, Fa. Seppic, FR) i.m. injiziert. Drei, fünf und sieben Wochen nach der Erstinjektion erfolgen Boosterinjektionen mit gleicher Menge. Eine Woche nach der letzten Injektion werden die Tiere getötet und ausgeblutet. Nachdem das Blut geronnen ist, wird das Antiserum durch Zentrifugation gewonnen und Natriumazid bis zu einer Endkonzentration von 0,02% zugefügt. Das Antiserum wird bei -20 °C eingefroren gelagert.

### Beispiel 12: Erzeugung von monoklonalen Antikörpern gegen das Peptid CysGlnGlnGlnThrAlaProLysAlaProThrGlu

Zwei Mäuse werden jeweils mit einer Emulsion aus 0,1 ml Konjugat aus Beispiel 10 und 0,1 ml Öladjuvans (MISA 50, Fa. Seppic, FR) s.c. injiziert. Zwei, vier und sechs Wochen nach der Erstinjektion erfolgen Boosterinjektionen mit gleicher Menge. Drei Tage nach der letzten Injektion werden die Tiere getötet und die Milz isoliert. Die Zellen aus der Milz werden nach üblichen Verfahren isoliert und mit einer permantenten murinen Zellinie fusioniert. Aus den Fusionsprodukten werden Zellinien selektioniert, die Antikörper gegen das Peptid CysGlnGlnGlnThrAlaProLysAlaProThrGlu bilden.

### Beispiel 13: Immunologischer Nachweis von L. monocytogenes

a) Vorkultur und Zentrifugation der Bakterien: 10 ml CASO-Bouillon werden mit Material aus mehreren Kolonien von L. monocytogenes angeimpft und bei 30°C über Nacht inkubiert. Anschließend wird je 1 ml der Kultur entnommen. Die Bakterienzellen werden abzentrifugiert (13000 UpM).
b) Identifizierungsreaktion: Je 300 µl der Überstände aus dem vorigen Arbeitsschritt werden in die Vertiefungen einer Mikrotiterplatte pipettiert und bei 4 °C über Nacht inkubiert. Anschließend wird je dreimal mit je 100 µl Waschlösung (9 g/l NaCl und 0,05% Tween 20 in Wasser) gewaschen. In die Vertiefungen werden nun je 100 µl Antiserum hergestellt nach Beispiel 11 pipettiert und eine Stunde bei Raumtemperatur inkubiert. Es wird erneut je dreimal mit je 100 µl Waschlösung gewaschen. Dann werden in jede Vertiefung je 100 µl mit alkalischer Phosphatase markierter anti-kaninchen Antikörperlösung (Art.Nr. A 8025; Fa. Sigma) pipettiert und 30 Minuten bei Raumtemperatur inkubiert. Die Vertiefungen werden erneut mit je 100 µl Waschlösung gewaschen und anschließend der gebundene enzymmarkierte Antikörper nachgewiesen. Dazu werden 200 µl einer Pufferlösung mit Substrat eingefüllt und 30 Minuten bei Raumtemperatur inkubiert. Die Reaktion wird durch Zugabe von je 100 µl 2 N NaOH-Lösung (Art.Nr. 9136, Fa. Merck) abgestoppt und das Reaktionsprodukt sichtbar gemacht. Eine gelborange Färbung zeigt die Anwesenheit von L. monocytogenes an.

### Beispiel 14: Spezifischer Nachweis von L. monocytogenes mittels Immunoblot

Bakterien werden wie in Beispiel 13a) beschrieben vorkultiviert und die Zellen abzentrifugiert. Die abzentrifugierten Zellen werden in 1 ml phosphatgepufferter Saline aufgenommen und suspendiert. 2 µl dieser Suspension werden auf eine Nitrocellulosemembran (Hybond C, 0,45 µm, Art.Nr. RPN 283 C, Fa. Amersham) pipettiert. Nachdem die Lösung eingetrocknet ist, wird die Membran für eine Stunde bei Raumtemperatur mit einer Lösung von Rinderserumalbumin (10 g/l) in phosphatgepufferter Saline behandelt. Es wird eine Verdünnung (1:200) des in Beispiel 11 erhaltenen Antiserums mit einer Lösung von Rinderserumalbumin (10 g/l) und Tween 20 (0,5 g/l) in phosphatgepufferter Saline (Antikörperlosung A), sowie eine weitere Verdünnung (1:500) von peroxidasemarkiertem anti-Kaninchen Antikörper (anti Rabbit IgG, Art.Nr. 68-397; Fa. ICN Immuno Biologicals) mit demselben Verdünnungsmittel (HRP-Antikörperlösung) vorbereitet. Die Membran wird eine Stunde bei Raumtemperatur mit Antikörperlösung A inkubiert und anschließend dreimal mit phosphatgepufferter Saline mit 0,05% Tween 20 gewaschen. Um die Antikörperbindung nachzuweisen, wird die Membran anschließend eine Stunde bei Raumtemperatur mit HRP-Antikörperlösung inkubiert und je dreimal mit a) Tween 20 (0.5 g/l) in phosphatgepufferter Saline, b) mit phosphatgepufferter Saline und c) mit TRIS-Puffer (50 mM; pH 7,4; mit 200 mM NaCl) gewaschen. Für die Farbreaktion wird eine Lösung von 4-Chloro-1-naphthol (3 mg/ml in Methanol) mit fünf Volumen TRIS-Puffer (50 mM; pH 7,4; mit 200 mM NaCl) verdünnt und Wasserstoffperoxid zugesetzt (Endkonzentration 0,1 g/l). Die Membran wird in dieser Substratlösung inkubiert. Eine blauschwarze Färbung zeigt L. monocytogenes an.

**Tabelle 1:**

| **Spezifität der Polymerase-Ketten-Reaktion bei Verwendung verschiedener Primer entsprechend Formel IIa - IIh** | | | | | | | | |
|---|---|---|---|---|---|---|---|---|
| Kombination: | A | B | C | D | E | F | G | H |
| Primer 1: | IId | IId | IIg | IIc | IIf | IIa | IIh | M³⁾ |
| Primer 2:¹) | IIe | IIb | IIb | IIb | IIb | IIb | IIb | IIb |
| | | | | | | | | |
| untersuchte Reaktion | | | | | | | | |
| Bakterien: | | | | | | | | |
| L. monocytogenes | | | | | | | | |
| Serovar 1/2a EGD | + | + | - | + | - | + | - | + |
| Serovar 1/2a Mack.²⁾ | + | + | - | + | - | + | - | + |
| Serovar 1/2b | + | + | - | + | - | + | - | + |
| Serovar 1/2c | + | + | - | + | - | + | - | + |
| Serovar 3a | + | + | - | + | - | + | - | + |
| Serovar 3b | + | + | - | + | - | + | - | + |
| Serovar 3c | + | + | - | + | - | + | - | + |
| Serovar 4a | + | + | - | + | - | + | - | + |
| Serovar 4ab | + | + | - | + | - | + | - | + |
| Serovar 4b | + | + | - | + | - | + | - | + |
| Serovar 4c | + | + | - | + | - | + | - | + |
| Serovar 4d | + | + | - | + | - | + | - | + |
| Serovar 4e | + | + | - | + | - | + | - | + |
| Serovar 7 | + | + | - | + | - | + | - | + |
| L. ivanovii | - | - | - | + | + | + | - | + |
| L. seeligeri | - | - | - | + | + | + | - | + |
| L. innocua | | | | | | | | |
| Serovar 6a | - | - | + | + | - | + | - | + |
| Serovar 6b | - | - | + | + | - | + | - | + |
| Serovar 4ab | - | - | + | + | - | + | - | + |
| L. welshimeri | - | - | - | + | + | + | - | + |
| L. murrayi | - | - | - | + | - | + | + | + |
| L. grayi | - | - | - | + | - | + | + | + |
| Enterococcus faecalis | - | - | - | - | - | + | - | - |
| Bacillus cereus | - | - | - | - | - | + | - | - |
| Micrococcus flavus | - | - | - | - | - | + | - | - |

| | | | | | | | | |
|---|---|---|---|---|---|---|---|---|
| Legende: + PCR-Produkt nachgewiesen - kein PCR-Produkt nachweisbar 1) komplementäre Sequenz | | | | | | | | |
| 2) Mack.: Stamm Mackaness | | | | | | | | |
| 3) M: Mischung aus Primern nach Formel IId, IIf, IIg und IIh; Amplifikationsprodukte können auf Grund des Molekulargewichtes differenziert werden. | | | | | | | | |

## Patentansprüche

1. Peptid, ausgewählt aus der Sequenz des Proteins p60 aus Bakterien der Art Listeria monocytogenes, **gekennzeichnet durch** eine Sequenz nach einer der Formeln IVa [SeqID 17] oder IVd [SeqID 20], worin
X³ und X⁴ jeweils unabhängig voneinander Wasserstoff, eine beliebige Aminosäure oder ein beliebiges Oligopeptid mit bis zu 7 Aminosäuren bedeuten.

2. Peptid nach Anspruch 1, **gekennzeichnet durch** eine der in [SeqID 26], [SeqID 29], [SeqID 30] oder [SeqID 31] dargestellten Sequenzen.

3. Verwendung eines Peptides nach einem der Ansprüche 1 - 2 zur Herstellung von immunogenen Konjugaten.

4. Antikörper gerichtet gegen das Protein p60 aus Listeria monocytogenes, **dadurch gekennzeichnet, daß** er ein Epitop bindet, welches eine der in [SeqID 26], [SeqID 29], [SeqID 30] oder [SeqID 31] dargestellten Sequenzen enthält.

5. Antikörper, herstellbar durch Immunisierung eines Versuchstieres mit einem immunogenen Konjugat, welches ein Peptid mit 7 bis 24 Aminosäuren enthält, **dadurch gekennzeichnet, daß** das Peptid eine der in [SeqID 17], [SeqID 20], [SeqID 26], [SeqID 29], [SeqID 30] oder [SeqID 31] dargestellten Sequenzen enthält.

6. Verfahren zur Herstellung eines Antikörpers gerichtet gegen das Protein p60 aus Listeria monocytogenes, indem man ein Versuchstier mit einem Immunogen immunisiert und den Antikörper isoliert, **dadurch gekennzeichnet, daß** man als Immunogen ein immunogenes Konjugat verwendet, das ein Peptid nach einer der Formeln IVa [SeqID 17] oder IVd [SeqID 20] aus Anspruch 1 enthält.

7. Verfahren nach Anspruch 6, **dadurch gekennzeichnet, daß** man als Immunogen ein immunogenes Konjugat verwendet, das ein Peptid nach einer der in [SeqID 26], [SeqID 29], [SeqID 30] oder [SeqID 31] dargestellten Sequenzen aus Anspruch 2 enthält.

8. Verwendung eines Antikörpers nach einem der Ansprüche 4 oder 5 für den Nachweis von Bakterien der Art Listeria monocytogenes.

9. Verfahren zum Nachweis von Bakterien der Art Listeria monocytogenes mittels einer Immunreaktion, **dadurch gekennzeichnet, daß** ein Antikörper nach einem der Ansprüche 4 oder 5 verwendet wird.

10. Testzusammenstellung zum Nachweis von Bakterien der Art Listeria monocytogenes mittels Immunoassay, **dadurch gekennzeichnet, daß** darin ein Antikörper nach einem der Ansprüche 4 oder 5 enthalten ist.

## Claims

1. Peptide which is selected from the sequence of the protein p60 from bacteria of the species Listeria monocytogenes, **characterized by** a sequence according to one of the formulae IVa[SeqID 17] or IVd[SeqID 20], in which
X³ and X⁴ are in each case, independently of each other, hydrogen, an arbitrary amino acid or an arbitrary oligopeptide having up to 7 amino acids.

2. Peptide according to Claim 1, **characterized by** one of the sequences depicted in [SeqID 26], [SeqID 29], [SeqID 30] or [SeqID 31].

3. Use of a peptide according to either Claim 1 or 2 for preparing immunogenic conjugates.

4. Antibody which is directed against the protein p60 from Listeria monocytogenes, **characterized in that** it binds an epitope which contains one of the sequences depicted in [SeqID 26], [SeqID 29], [SeqID 30] or [SeqID 31].

5. Antibody which can be prepared by immunizing an experimental animal with an immunogenic conjugate which contains a peptide having from 7 to 24 amino acids,
**characterized in that** the peptide contains one of the sequences depicted in [SeqID 17], [SeqID 20] [SeqID 26], [SeqID 29], [SeqID 30] or [SeqID 31].

6. Process for preparing an antibody which is directed against the protein p60 from Listeria monocytogenes by immunizing an experimental animal with an immunogen and isolating the antibody, **characterized in that** the immunogen employed is an immunogenic conjugate which contains a peptide according to one of the formulae IVa[SeqID 17] or IVd[SeqID 20] from Claim 1.

7. Process according to Claim 6, **characterized in that** the immunogen employed is an immunogenic conjugate which contains a peptide according to one of the sequences depicted in [SeqID 26], [SeqID 29], [SeqID 30] or [SeqID 31] from Claim 2.

8. Use of an antibody according to one of Claims 4 or 5 for detecting bacteria of the species Listeria monocytogenes.

9. Process for detecting bacteria of the species Listeria monocytogenes by means of an immune reaction, **characterized in that** use is made of an antibody according to one of Claims 4 or 5.

10. Test compilation for detecting bacteria of the species Listeria monocytogenes by means of immunoassay, **characterized in that** the compilation contains an antibody according to one of Claims 4 or 5.

## Revendications

1. Peptide choisi parmi la séquence de la protéine p60 provenant de bactéries appartenant à l'espèce *Listeria monocytogenes*, **caractérisé par** une séquence selon l'une des formules IVa [SEQ ID 17] et IVd [SEQ ID 20] dans lesquelles
X³ et X⁴ représentent chacun, indépendamment l'un de l'autre, un atome d'hydrogène, un aminoacide quelconque ou un oligopeptide quelconque comportant jusqu'à 7 aminoacides.

2. Peptide selon la revendication 1, **caractérisé par** l'une des séquences représentées dans [SEQ ID 26], [SEQ ID 29], [SEQ ID 30] ou [SEQ ID 31].

3. Utilisation d'un peptide selon l'une quelconque des revendications 1 à 2, pour la production de conjugués immunogènes.

4. Anticorps dirigé contre la protéine p60 de *Listeria monocytogenes,* **caractérisé en ce qu'**il se lie à un épitope qui contient l'une des séquences représentées dans [SEQ ID 26], [SEQ ID 29], [SEQ ID 30] ou [SEQ ID 31].

5. Anticorps pouvant être produit par immunisation d'un animal expérimental avec un conjugué immunogène qui contient un peptide comportant de 7 à 24 aminoacides, **caractérisé en ce que** le peptide contient l'une des séquences représentées dans [SEQ ID 17], [SEQ ID 20], [SEQ ID 26], [SEQ ID 29], [SEQ ID 30] ou [SEQ ID 31].

6. Procédé pour la production d'un anticorps dirigé contre la protéine p60 provenant de *Listeria monocytogenes*, par immunisation d'un animal expérimental avec une substance immunogène et isolement de l'anticorps, **caractérisé en ce qu'**on utilise comme substance immunogène un conjugué immunogène qui contient un peptide selon l'une des formules IVa [SEQ ID 17] et IVd [SEQ ID 20] de la revendication 1.

7. Procédé selon la revendication 6, **caractérisé en ce qu'**on utilise comme substance immunogène un conjugué immunogène qui contient un peptide selon l'une des séquences représentées dans [SEQ ID 26], [SEQ ID 29], [SEQ ID 30] ou [SEQ ID 31] de la revendication 2.

8. Utilisation d'un anticorps selon la revendication 4 ou 5, pour la détection de bactéries appartenant à l'espèce *Listeria monocytogenes.*

9. Procédé pour la détection de bactéries appartenant à l'espèce *Listeria monocytogenes* au moyen d'une réaction immunologique, **caractérisé en ce qu'**on utilise un anticorps selon la revendication 4 ou 5.

10. Composition d'essai pour la détection de bactéries appartenant à l'espèce *Listeria monocytogenes* au moyen d'un dosage immunologique, **caractérisée en ce qu'**un anticorps selon la revendication 4 ou 5 est contenu dans celle-ci.


### SEQUENCE LISTING

(1) GENERAL INFORMATION:
   (i) APPLICANT:
      (A) NAME: Merck Patent GmbH
      (B) STREET: Frankfurter Str. 250
      (C) CITY: Darmstadt
      (E) COUNTRY: Germany
      (F) POSTAL CODE (ZIP): 64271
      (G) TELEPHONE: 06151-727840
      (H) TELEFAX: 06151-727191
      (I) TELEX: 4 19 328 32 em d
   (ii) TITLE OF INVENTION: Verfahren und Mittel zum Nachweis von Listerien
   (iii) NUMBER OF SEQUENCES: 40
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: EP 93108775.3
   (vi) PRIOR APPLICATION DATA:
      (A) APPLICATION NUMBER: DE 4219111
      (B) FILING DATE: 11-JUN-1992
   (vi) PRIOR APPLICATION DATA:
      (A) APPLICATION NUMBER: DE 4239567
      (B) FILING DATE: 25-NOV-1992
(2) INFORMATION FOR SEQ ID NO:1:
   (i) SEQUENCE CHARACTERISTICS:
      (A) LENGTH: 14 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESORIPTION: SEQ ID NO:1:
(2) INFORMATION FOR SEQ ID NO:2:
   (i) SEQUENCE CHARACTERISTICS:
      (A) LENGTH: 14 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:2:
   GCTTCGGTCG CGTA 14
(2) INFORMATION FOR SEQ ID NO:3:
   (i) SEQUENCE CHARACTERISTICS:
      (A) LENGTH: 14 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:3:
   ACAGCTGGGA TTGC 14
(2) INFORMATION FOR SEQ ID NO:4:
   (i) SEQUENCE CHARACTERISTICS:
      (A) LENGTH: 15 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:4:
   ACTGCTAACA CAGCT 15
(2) INFORMATION FOR SEQ ID NO:5:
   (i) SEQUENCE CHARACTERISTICS:
      (A) LENGTH: 15 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:5:
   TAACAGCAAT TCAAG 15
(2) INFORMATION FOR SEQ ID NO:6:
   (i) SEQUENCE CHARACTERISTICS:
      (A) LENGTH: 14 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:6:
   CTGAGGTAGC GAGC 14
(2) INFORMATION FOR SEQ ID NO:7:
   (i) SEQUENCE CHARACTERISTICS:
      (A) LENGTH: 16 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:7:
   AGCACTCCAG TTGTTA 16
(2) INFORMATION FOR SEQ ID NO:8:
   (i) SEQUENCE CHARACTERISTICS:
      (A) LENGTH: 15 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:8:
   GCAGTTTCTA AACCT 15
(2) INFORMATION FOR SEQ ID NO:9:
   (i) SEQUENCE CHARACTERISTICS:
      (A) LENGTH: 25 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:9:
   GTCGACTGAA TATGAAAAAA GCAAC 25
(2) INFORMATION FOR SEQ ID NO:10:
   (i) SEQUENCE CHARACTERISTICS:
      (A) LENGTH: 26 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:10:
   TTGGCTTCGG TCGCGTAGAA TTCATA 26
(2) INFORMATION FOR SEQ ID NO:11:
   (i) SEQUENCE CHARACTERISTICS:
      (A) LENGTH: 20 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:11:
   GCTACAGCTG GGATTGCGGT 20
(2) INFORMATION FOR SEQ ID NO:12:
   (i) SEQUENCE CHARACTERISTICS:
      (A) LENGTH: 21 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:12:
   CAAACTGCTA ACACAGCTAC T 21
(2) INFORMATION FOR SEQ ID NO:13:
   (i) SEQUENCE CHARACTERISTICS:
      (A) LENGTH: 21 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:13:
   CAATAACAGC AATTCAAGTG C 21
(2) INFORMATION FOR SEQ ID NO:14:
   (i) SEQUENCE CHARACTERISTICS:
      (A) LENGTH: 20 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:14:
   TAACTGAGGT AGCGAGCGAA 20
(2) INFORMATION FOR SEQ ID NO:15:
   (i) SEQUENCE CHARACTERISTICS:
      (A) LENGTH: 22 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO: 15:
   ACTAGCACTC CAGTTGTTAA AC 22
(2) INFORMATION FOR SEQ ID NO:16:
   (i) SEQUENCE CHARACTERISTICS:
      (A) LENGTH: 21 base pairs
      (B) TYPE: nucleic acid
      (C) STRANDEDNESS: double
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Listeria monocytogenes
   (xi) SEQUENCE DESCRIPTION: SEQ ID NO:16:
   CCAGCAGTTT CTAAACCTGC T 21
(2) INFORMATION FOR SEQ ID NO:17:
   (i) SEQUENCE CHARACTERISTICS:
      (A) LENGTH: 6 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:17:
   Pro Val Ala Pro Thr Gln
   1 5
(2) INFORMATION FOR SEQ ID NO:18:
   (i) SEQUENCE CHARACTERISTICS:
      (A) LENGTH: 7 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:18:
   Thr Gln Ala Thr Thr Pro Ala
   1 5
(2) INFORMATION FOR SEQ ID NO:19:
   (i) SEQUENCE CHARACTERISTICS:
      (A) LENGTH: 9 amino acids
      (8) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:19:
   Ala Ile Lys Gln Thr Ala Asn Thr Ala
   1               5
(2) INFORMATION FOR SEQ ID NO:20:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:20:
   Gln Gln Thr Ala Pro Lys Ala Pro Thr
   1               5
(2) INFORMATION FOR SEQ ID NO:21:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:21:
   Val Asn Asn Glu Val Ala Ala Ala Glu Lys Thr Glu
   1               5                   10
(2) INFORMATION FOR SEQ ID NO:22:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:22:
   Thr Pro Val Val Lys Gln Glu Val Lys
   1               5
(2) INFORMATION FOR SEQ ID NO:23:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:23:
   Val Lys Gln Pro Thr Thr Gln Gln Thr Ala Pro
   1               5                   10
(2) INFORMATION FOR SEQ ID NO:24:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:24:
   Ile Lys Gln Pro Thr Lys Thr Val Ala Pro
   1               5                   10
(2) INFORMATION FOR SEQ ID NO:25:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:25:
   Gln Gln Thr Thr Thr Lys Ala Pro Thr
   1               5
(2) INFORMATION FOR SEQ ID NO:26:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:26:
   Val Ser Thr Pro Val Ala Pro Thr Gln
   1               5
(2) INFORMATION FOR SEQ ID NO:27:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:27:
   Thr Thr Gln Ala Thr Thr Pro Ala Pro Lys Val Ala
   1               5                   10
(2) INFORMATION FOR SEQ ID NO:28:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:28:
   Leu Ala Ile Lys Gln Thr Ala Asn Thr Ala Thr
   1               5                   10
(2) INFORMATION FOR SEQ ID NO:29:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:29:
   Gln Gln Gln Thr Ala Pro Lys Ala Pro Thr Glu
   1               5                   10
(2) INFORMATION FOR SEQ ID NO:30:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:30:
   Ser Thr Pro Val Ala Pro Thr Gln Glu Val Lys Lys
   1               5                   10
(2) INFORMATION FOR SEQ ID NO:31:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:31:
   Pro Val Ala Pro Thr Gln Glu Val Lys Lys
   1               5                   10
(2) INFORMATION FOR SEQ ID NO:32:
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:32:
   Gln Val Asn Asn Glu Val Ala Ala Ala Glu Lys Thr Glu Lys
   1               5                   10
(2) INFORMATION FOR SEQ ID NO:33:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:33:
   Glu Val Lys Gln Thr Thr Gln Ala Thr Thr Pro Ala
   1 5 10
(2) INFORMATION FOR SEQ ID NO:34:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:34:
   Ala Ile Lys Gln Thr Ala Asn Thr Ala Thr Pro Lys
   1 5 10
(2) INFORMATION FOR SEQ ID NO:35:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:35:
   Ser Thr Pro Val Val Lys Gln Glu Val Lys Lys
   1 5 10
(2) INFORMATION FOR SEQ ID NO:36:
   (i) SEQUENCE CHARACTERISTICS:
      (A) LENGTH: 13 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:36:
   Glu Val Lys Gln Pro Thr Thr Gln Gln Thr Ala Pro Ala
   1 5 10
(2) INFORMATION FOR SEQ ID NO:37:
   (i) SEQUENCE CHARACTERISTICS:
      (A) LENGTH: 12 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:37:
   Ala Ile Lys Gln Pro Thr Lys Thr Val Ala Pro Lys
   1 5 10
(2) INFORMATION FOR SEQ ID NO:38:
   (i) SEQUENCE CHARACTERISTICS:
      (A) LENGTH: 11 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria innocua
   (xi) SEQUENCE DESCRIPTION: SEQ ID NO:38:
   Glu Gln Gln Thr Thr Thr Lys Ala Pro Thr Gln
   1 5 10
(2) INFORMATION FOR SEQ ID NO:39:
   (i) SEQUENCE CHARACTERISTICS:
      (A) LENGTH: 232 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:39:
   Gln Val Asn Asn Glu Val Ala Ala Ala Glu Lys Thr Glu Lys Ser Val
   1               5                   10                  15
   Ser Ala Thr Trp Leu Asn Val Arg Thr Gly Ala Gly Val Asp Asn Ser
               20                  25                  30
   Ile Ile Thr Ser Ile Lys Gly Gly Thr Lys Val Thr Val Glu Thr Thr
           35                  40                  45
   Glu Ser Asn Gly Trp His Lys Ile Thr Tyr Asn Asp Gly Lys Thr Gly
       50                  55                  60
   Phe Val Asn Gly Lys Tyr Leu Thr Asp Lys Ala Val Ser Thr Pro Val
   65                  70                  75                  80
   Ala Pro Thr Gln Glu Val Lys Lys Glu Thr Thr Thr Gln Gln Ala Ala
                   85                  90                  95
   Pro Val Ala Glu Thr Lys Thr Glu Val Lys Gln Thr Thr Gln Ala Thr
               100                 105                 110
   Thr Pro Ala Pro Lys Val Ala Glu Thr Lys Glu Thr Pro Val Ile Asp
           115                 120                 125
   Gln Asn Ala Thr Thr His Ala Val Lys Ser Gly Asp Thr Ile Trp Ala
       130                 135                 140
   Leu Ser Val Lys Tyr Gly Val Ser Val Gln Asp Ile Met Ser Trp Asn
   145                 150                 155                 160
   Asn Leu Ser Ser Ser Ser Ile Tyr Val Gly Gln Lys Leu Ala Ile Lys
                   165                 170                 175
   Gln Thr Ala Asn Thr Ala Thr Pro Lys Ala Glu Val Lys Thr Glu Ala
               180                 185                 190
   Pro Ala Ala Glu Lys Gln Ala Ala Pro Val Val Lys Glu Asn Thr Asn
           195                 200                 205
   Thr Asn Thr Ala Thr Thr Glu Lys Lys Glu Thr Ala Thr Gln Gln Gln
       210                 215                 220
   Thr Ala Pro Lys Ala Pro Thr Glu
   225                 230
(2) INFORMATION FOR SEQ ID NO:40:
   (i) SEQUENCE CHARACTERISTICS:
      (A) LENGTH: 478 amino acids
      (B) TYPE: amino acid
      (D) TOPOLOGY: linear
   (ii) MOLECULE TYPE: peptide
   (vi) ORIGINAL SOURCE:
      (A) ORGANISM: Listeria monocytogenes
   (xi) SEQUENCE DESCRIPTION: SEQ ID NO:40: